# EUROPEAN PATENT SPECIFICATION

(11) **EP 1 899 310 B1**
(45) Date of publication and mention of the grant of the patent: **29.09.2010**
(21) Application number: 06779787.8
(22) Date of filing: 12.06.2006
(51) Int. Cl.: C07D 295/08

(54) **STEREOSELECTIVE HYDROGENATION PROCESS FOR PREPARING CIS-6-PHENYL-5-[4-(2-PYRROLIDIN-1-YL-ETHOXY)-PHENYL]-2-METHOXY-5,6,7,8-TETRAHYDRONAPHTHALENE HYDROCHLORIDE**
STEREOSELEKTIVES HYDRIERVERFAHREN ZUR HERSTELLUNG VON CIS-6-PHENYL-5-[4-(2-PYRROLIDIN-1-YL-ETHOXY)-PHENYL]-2-METHOXY-5,6,7,8-TETRAHYDRONAPHTHALIN-HYDROCHLORID
PROCEDE D'HYDROGENATION STEREOSELECTIVE LA PREPARATION DE CHLORHYDRATE DE CIS-6-PHENYL-5-[4-(2-PYRROLIDIN-1-YL-ETHOXY)-PHENYL]-2-METHOXY-5,6,7,8-TETRAHYDRONAPHTALENE

(30) Priority: 22.06.2005 US 692882 P
(43) Date of publication of application: 19.03.2008
(73) Proprietor: Pfizer Products Inc., Groton, CT 06340 (US)
(72) Inventor: LEHNER, Richard Shelton Pfizer Global Res. & Dvlp., Groton, CT 06340 (US); TABER, Geraldine P. Pfizer Global Research & Dvlp., Groton, CT 06340 (US)
(74) Representative: Dekker, Henrike Cornelie Christine
(86) International application number: PCT/IB2006/001773
(87) International publication number: WO 2006/136944

(56) References cited:
- WO-A-96/21656
- US-A- 5 948 809
- ROSATI R L ET AL: "DISCOVERY AND PRECLINICAL PHARMACOLOGY OF A NOVEL POTENT NONSTEROIDAL ESTROGEN RECEPTOR AGONIST/ANTAGONIST CP-336156 A DIARYLTETRAHYDRONAPHTHALENE" JOURNAL OF MEDICINAL CHEMISTRY, AMERICAN CHEMICAL SOCIETY. WASHINGTON, US, vol. 41, no. 16, 1998, pages 2928-2931, XP000891443 ISSN: 0022-2623

## Description

### Background of the Invention

This invention provides a process for preparing cis-6-phenyl-5-[4-(2-pyrrolidin-1-yl-ethoxy)phenyl]-2-methoxy-5,6,7,8-tetrahydronaphthalene hydrochloride which is an intermediate in the synthesis of (-)-cis-6-phenyl-5-[4-(2-pyrrolidin-1-yl-ethoxy)-phenyl]-5,6,7,8-tetrahydronaphthalene-2-ol.

A preparation of (-)-cis-6-phenyl-5-[4-(2-pyrrolidin-1-yl-ethoxy)-phenyl]-5,6,7,8-tetrahydronaphthhalene-2-ol and salts thereof, also known as lasofoxifene, is described in U.S. Patent 5,552,412. Lasofoxifene is the active ingredient in Oporia®, which is currently undergoing regulatory review. This compound is a selective estrogen receptor modulator and is useful for treatment and prevention of physiological disorders associated with menopause such as osteoporosis, vaginal atrophy, female sexual dysfunction, cardiovascular disease and breast cancer. U.S. Patent 5,552,412 also describes the synthesis of cis-1-{2-[4-(6-methoxy-2-phenyl-1,2,3,4-tetrahydronaphthalen-1-yl)phenoxy]ethyl}pyrrolidine by hydrogenation of nafoxidine. The preparation of (-)cis-6-phenyl-5-[4-(2-pyrrolidin-1-yl-ethoxy)-phenyl]-5,6,7,8-tetrahydronaphthalene-2-ol, D-tartrate is described in U.S. Patent 5,948,809.

The hydrogenation of nafoxidine using Pd(OH)₂/C is described in general terms in J. Med. Chem., 1998, 41, 2928-2931.

### Summary of the Invention

This invention provides a process for preparing cis-6-phenyl-5-[4-(2-pyrrolidin-1-yl-ethoxy)-phenyl]-2-methoxy-5,6,7,8-tetrahydronaphthalene hydrochloride by the stereoselective hydrogenation of nafoxidine hydrochloride. This hydrogenation process maximizes the conversion of nafoxidine hydrochloride to cis-6-phenyl-5-[4-(2-pyrrolidin-1-yl-ethoxy)-phenyl]-2-methoxy-5,6,7,8-tetrahydronaphthalene hydrochloride while minimizing the amount of the aromatized impurity 1-{2-[4-(6-methoxy-2-phenyl-naphthalen-1-yl)-phenoxy]-ethyl}-pyrrolidine hydrochloride produced. The depiction of the conversion of nafoxidine hydrochloride (compound I) to cis-6-phenyl-5-[4-(2-pyrrolidin-1-yl-ethoxy)-phenyl]-2-methoxy-5,6,7,8-tetrahydronaphthalene hydrochloride (compound II) and the aromatized impurity (compound III) is shown in Reaction Scheme 1 below.

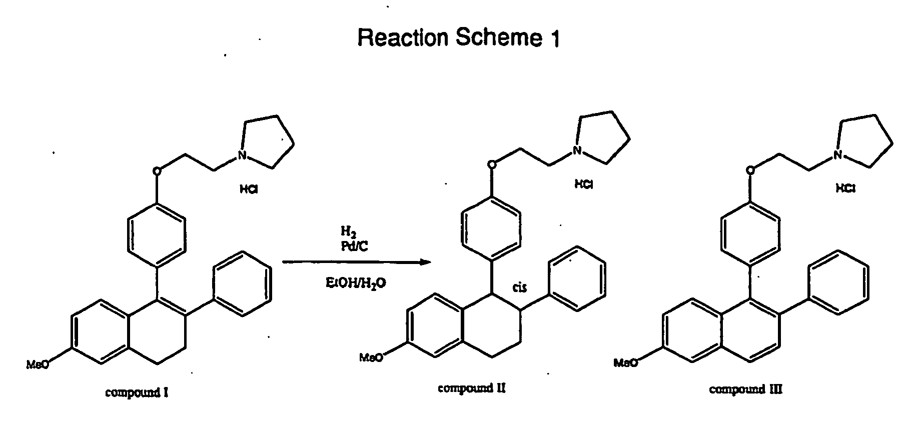

### Detailed Description of the Invention

The present invention provides a hydrogenation process for preparing cis-6-phenyl-5-[4-(2-pyrrolidin-1-yl-ethoxy)-phenyl]-2-methoxy-5,6,7,8-tetrahydronaphthalene hydrochloride (hereinafter compound II) that maximizes the conversion of nafoxidine hydrochloride (hereinafter compound I) to the desired product while minimizing the amount of the aromatized impurity, 1-{2-[4-(6-methoxy-2-phenyl-naphthalen-1-yl)-phenoxy]-ethyl}-pyrrolidine (hereinafter compound III) produced. Compound I is 1-{2-[4-(6-methoxy-2-phenyl-3,4-dihydronaphthalene-1-yl)phenoxy]ethyl}pyrrolidine hydrochloride and is a compound known in the art and can be prepared by known methods, including methods described in U.S. Patent No. 3,277,106.

Numerous reaction condition parameters were explored to determine what reaction conditions favor the undesired formation of compound III when preparing compound II by hydrogenating compound I. Compound **III** is useful as an analytical standard for determining the purity of comound II. Table I, below, provides a listing of reaction parameters and specific reaction conditions that were investigated as well as the percentage of compound III and percentage of total impurities that were formed using those reaction conditions. All reaction parameters and conditions not specified remain substantially as in the standard hydrogenation reaction conditions as provided in Example 1, hereinbelow.

The reaction parameters 1 a-14b explored for the present hydrogenation process are provided in Table I below. The first three entries in Table I were carried out substantially according to the general procedure as described in Example 1. Parameters 1 a-14b were explored by carrying out the procedure substantially according to the general procedure as described in Example 1, with the exception of a change in either or both of the parameter description and reaction condition as specified in columns 2 and 3 of the table. For example, parameter 1a was explored by carrying out the procedure of Example 1 with the exception that 9 mL of 2B ethanol per gram of compound was used instead of the standard 13.5 mL/g as in Example 1. The reactions exploring parameters 1a-14b were typically carried out on a 15 g or 20 g scale based on the amount of the compound I starting material. The percentage of compound III and the percentage of total impurities were determined by HPLC according to a standard HPLC protocol.

The standard HPLC analytical method employed a suitable liquid chromatograph equipped with a reverse phase 25 cm x 4.6 mm Symmetry^{®} C18 column (Waters Corporation, 34 Maple Street, Milford, Massachusetts 01757 USA), a UV detector capable of monitoring at 230 nm and an injector or auto-sampler capable of making 50 □L injections. Gradient elution was employed using a mixture of water, trifluoroacetic acid and ammonium hydroxide (adjusted to pH 3 with ammonium hydroxide) as mobile phase A and a mixture of water, acetonitrile, trifluoroacetic acid and ammonium hydroxide (adjusted to pH 3 with ammonium hydroxide) as mobile phase B. The retention time and relative retention time, Rᵣ, of compound III was approximately 22 minutes and approximately 0.96, respectively, when compared with compound II which had a retention time of 23 minutes and an Rᵣ of approximately 1.00.

**Table I**

| Parameter | Parameter Description | Condition | Product Yield (%) | Compound III (% w/w) | Total Impurities (%w/w) |
|---|---|---|---|---|---|
| Standard | Standard | Standard | 93.7 | 1.9 | 2.0 |
| Standard | Standard | Standard | 95.2 | 2.4 | 2.4 |
| Standard | Standard | Standard | 93.5 | 2.4 | 2.6 |
| 1a | 2B Ethanol | 9 mL/g | 94.6 | 1.9 | 2.1 |
| 1b | 2B Ethanol | 16 mUg | 92.2 | 2.3 | 2.4 |
| 2a | Water | 1.0 mL/g | 95.1 | 2.1 | 2.2 |
| 2b | Water | 2.0 mUg | 90.3 | 1.4 | 1.5 |
| 3a | 2B ethanol Water | 9 mUg 2.0 mUg | 94.9 | 2.4 | 2.7 |
| 3b | 2B ethanol Water | 16 mUg 2.0 mUg | 93.0 | 1.3 | 1.4 |
| 4a | Catalyst charge | 0.05 g/g | 96.4 | 1.5 | 1.6 |
| 4b | Catalyst charge | 0.5 g/g | 92.2 | 1.9 | 2.6 |
| 5a | Hydrogenation Pressure | Ambient | 95.0 | 8.8 | 9.0 |
| 5b | Hydrogenation Pressure | 10 psi (68.95 kPa) | 95.2 | 3.9 | 4.0 |
| 5c | Hydrogenation Pressure | 20 psi (137.9 kPa) | 93.8 | 2.2 | 2.3 |
| 5d | Hydrogenation Pressure | 60 psi (413.7 kPa) | 94.3 | 1.8 | 1.9 |
| 6a | Hydrogenation Time | 2 Hours | 91.5 | 2.8 | 2.9 |
| 6b | Hydrogenation Time | 3 Hours | 94.1 | 1.3 | 1.4 |
| 6c | Hydrogenation Time | 48 Hours | 93.2 | 0.6 | 1.4 |
| 6d | Hydrogenation Time | 48 Hours | 95.6 | 1.1 | 2.1 |
| 7a | Hydrogenation Temperature | 40°C | 95.6 | 1.7 | 1.8 |
| 7b | Hydrogenation Temperature | 60°C | 92.7 | 3.0 | 3.5 |
| 8a | Volume of Ethanol Concentrate | 1.0 mUg | 93.0 | 2.9 | 3.0 |
| 8b | Volume of Ethanol Concentrate | 5.0 mUg | 94.5 | 2.7 | 2.8 |
| 9a | Volume Added Toluene | 10.0 mUg | 97.2 | 2.4 | 2.6 |
| 9b | Volume Added Toluene | 30 mL/g | 92.5 | 2.6 | 2.8 |
| 10a | Volume of Toluene Concentrate | 2 mUg | 93.1 | 2.8 | 3.1 |
| 10b | Volume of Toluene Concentrate | 5 mUg | 93.0 | 1.8 | 1.8 |
| 10c | Volume of Toluene Concentrate | 8 mUg | 94.6 | 1.6 | 1.7 |
| 11 | Granulation Time | 72 Hours | 96.5 | 3.4 | 3.6 |
| 12a | Granulation Temperature | 0*C to 5°C | 95.9 | 2.8 | 3.0 |
| 12b | Granulation Temperature | 30°C | 94.3 | 2.5 | 2.6 |
| 13 | Drying Temperature (no vacuum) | 45°C | 93.4 | 3.3 | 3.4 |
| 14a | Drying Time at 60°C | 5 Hours | 89.0 | 0.5 | 0.5 |
| 14b | Drying Time at 60°C | 48 Hours | 88.3 | 0.5 | 0.5 |

When exploring the reaction parameters 1a-14b, it was found that the percentage of compound III being formed correlated with parameters involving the reaction temperature and the hydrogenation pressure. Particularly, the amount of compound III formed increased with decreased hydrogenation pressure and higher reaction temperature as seen for parameters 5a-5d and 7a-7b in Table I.

Further investigation determined compound III is formed when the reaction mixture is at lower hydrogenation pressure and elevated temperatures, as shown below in Table II.

**Table II**

| Hydrogenation Reaction Pressure at 45-55 °C | 0 Atmospheres (0 kPa) | 0.681 atmospheres (69.0 kPa) | 1.36 atmospheres (137.8 kPa) | 3.07 atmospheres (311.0 kPa) |
|---|---|---|---|---|
| Amount of Compound III in Reaction | 18-24% | 8% | 2-3% | <2% |

As shown in Table II, when the reaction mixture is heated at 45 °C to 55 °C at 0 atmospheres hydrogen pressure (0 kPa) compound III is present between 18% to 24% of the total reaction yield. When the hydrogenation reaction is first pressurized to 0.681 atmospheres (69.0 kPa) and then heated to 45 °C to 55 °C the amount of compound III decreased to about 8% of the total reaction yield. The trend of decreasing amounts of compound III with increasing hydrogenation pressure prior to heating the reaction mixture continued as higher pressures were applied before heating the reaction mixture. When the hydrogenation reaction is first pressurized to 1.36 atmospheres (137.8 kPa) or 3.07 atmospheres (311.0 kPa) and then heated to 45 °C to 55 °C the amount of compound III decreased to about 2% to 3% and less than 2%, respectively, of the total reaction yield.

In a typical hydrogenation reaction that requires both heat and pressure, the reaction vessel is charged with an appropriate catalyst, starting material and solvent system and the system is heated prior to pressurizing it with hydrogen. Heating the reaction mixture prior to pressurizing it with hydrogen is standard practice because this allows for easier equilibration and monitoring of the reaction pressure. Compound I can not routinely be converted to compound II with less than 2.5 % of compound III present by way of the standard hydrogenation sequence of heating the hydrogenation reaction mixture prior to pressurizing with hydrogen. Instead, to obtain compound II with less than 2.5 % of compound III present the reaction mixture must be pressurized with hydrogen prior to the application of heat and the pressure has to be continually adjusted as increasing temperature elevates the pressure of the closed system. This sequence of carrying out the hydrogenation effectively minimizes the formation of compound III.

For the purposes of this hydrogenation, it was found that optimal hydrogen pressures for the minimization of compound III are in the range of about 1.36 atmospheres to about 6.80 atmospheres (about 20 psi (137.9 kPa) to about 100 psi (689.4 kPa)), preferably in the range of about 3.07 atmospheres to about 3.75 atmospheres (about 45 psi (310.2 kPa) to about 55 psi (379.2 kPa)). After pressurizing the reaction mixture under hydrogen, the hydrogenation reaction mixture is heated to a temperature of 45°C to 55°C. Heating the hydrogenation reaction mixture using the described sequence ensures that the hydrogenation converts about 98% of compound I to the desired compound II.

Any suitable solvent or solvent system can be used for the purposes of this hydrogenation. Although not required, a polar, protic solvent or solvent system is preferred for reasons of solubility. Appropriate solvents that can be used alone or as part of a solvent system for this hydrogenation include methanol, ethanol, propanol, isopropanol, tetrahydrofuran and water. A particular solvent system useful for this hydrogenation is a mixture of ethanol and water, more particularly a 9:1 mixture of ethanol and water. The concentration of the reaction mixture was not found to be critical, but this hydrogenation is typically run at a concentration of about 0.17 M with respect to the starting material compound I.

Any suitable hydrogenation catalyst can be used for the purposes of this hydrogenation. Examples of hydrogenation catalysts that can be used include palladium on carbon, palladium hydroxide, palladium on alumina, palladium on calcium carbonate, nickel catalysts, rhodium on carbon, platinum on carbon, chlorotris(triphenylphosphine)rhodium(I), and dichlorotris(triphenylphosphine) ruthenium(II). A preferred catalyst for this hydrogenation is palladium on carbon and more particularly 5% palladium on activated carbon.

The present hydrogenation process can be carried out for several hours to several days, however the optimal yield of compound II is attained in about 4 to 8 hours. When analysis of the hydrogenation reaction mixture by LCMS shows less than 1% of compound I starting material remaining the reaction is considered complete. The reaction mixture is then cooled, vented to remove the hydrogen and purged with nitrogen. The reaction mixture is then filtered through a filter aid such as a diatomaceous earth (Celite 545^{®}, Mallinckrodt Baker, Inc., Phillipsburg, NJ 08865, USA) to remove the catalyst. The hydrogenation reaction vessel and filter cake are then washed with an appropriate solvent such as 2B ethanol and the combined filtrate is concentrated under vacuum at about 30 °C to 50 °C. The concentrate is then azeotroped with toluene and concentrated under vacuum at about 30 °C to 50 °C again to a concentration of about 2.7 mUg based on the amount of compound I originally used. The resulting slurry is cooled to about 20 °C and stirred for about 2 hours. The product is then collected by filtration and washed with toluene then dried under vacuum at about 40 °C to 50 °C until a constant weight is achieved. The resulting compound II is typically in a mean yield of about 97.5% with 2.2 ± 0.3% of total impurities.

The present invention provides a specific hydrogenation process for preparing cis-6-phenyl-5-[4-(2-pyrrolidin-1-yl-ethoxy)-phenyl]-2-methoxy-5,6,7,8-tetrahydronaphthalene hydrochloride, wherein said cis-6-phenyl-5-[4-(2-pyrrolidin-1-yl-ethoxy)phenyl]-2-methoxy-5,6,7,8-tetrahydronaphthalene hydrochloride contains less than 2.5% of 1-{2-[4-(6-methoxy-2-phenyl-naphthalen-1-yl)phenoxy]-ethyl}-pyrrolidine hydrochloride, the process comprising, in the following order, the steps of:
(a) charging a hydrogenation reaction vessel with an appropriate hydrogenation catalyst and a solution of 1-{2-[4-(6-methoxy-2-phenyl-3,4-dihydronaphthalene-1-yl)phenoxy]ethyl}pyrrolidine hydrochloride in an appropriate solvent;
(b) pressurizing the hydrogenation reaction vessel with hydrogen to a pressure of 137.9 to 689.4 kPa (20 to 100 psi) to provide a hydrogenation reaction mixture;
(c) heating the hydrogenation reaction mixture of step (b) at a temperature of 45°C to 55°C until 1% or less of the 1-{2-[4-(6-methoxy-2-phenyl-3,4-dihydronaphthalene-1-yl)phenoxy]ethyl}pyrrolidine hydrochloride remains to provide cis-6-phenyl-5-[4-(2-pyrralidin-1-yl-ethoxy)-phenyl]-2-methoxy-5,6,7,8-tetrahydronaphthalene hydrochloride;
(d) isolating the resulting cis-6-phenyl-5-[4-(2-pyrrotidin-1-yl-ethoxy)-phenyl]-2-methoxy-5,6,7,8-tetrahydronaphthalene hydrochloride; wherein the resulting cis-6-phenyl-5-[4-(2-pyrrolidin-1-yl-ethoxy)-phenyl]-2-methoxy-5,6,7,8-tetrahydronaphthalene hydrochloride contains less than 2.5% of 1-{2-[4-(6-methoxy-2-phenyl-naphthalen-1-yl)-phenoxy]-ethyl}-pyrrolidine hydrochloride.

In the present invention the pressure range in step (b) of the hydrogenation process is 20 psi (137.9 kPa) to 100 psi (689.4 kPa), with a preferred pressure in step (b) being 20 psi (137.9 kPa) to 60 psi (413.6 kPa), and a more preferred pressure range in step (b) being 45 psi (310.2 kPa) to 55 psi (379.2 kPa). The temperature range in step (c) of the hydrogenation process is 45 °C to 55 °C. A preferred process of this invention is one wherein the pressure in step (b) is 45 psi (310.2 kPa) to 55 psi (379.2 kPa) and the temperature in step (c) is 45 °C to 55 °C. In a further embodiment of this invention the hydrogenation catalyst in step (a) of the process is palladium on carbon and the solvent in step (a) is a mixture of ethanol and water. A preferred embodiment of the invention is one wherein the hydrogenation catalyst is 5% palladium on activated carbon. Another preferred embodiment of the invention is one wherein the solvent in step (a) is a 9:1 mixture of ethanol to water. Yet another preferred embodiment of the invention is the process wherein the hydrogenation catalyst in step (a) is 5% palladium on activated carbon and the solvent in step (a) is a 9:1 mixture of ethanol to water, the pressure in step (b) is 45 psi (310.2 kPa) to 55 psi (379.2 kPa) and the temperature in step (c) is 45 °C to 55 °C.

Abbreviations used in this description are defined as follows:

| | |
|---|---|
| g | gram(s) |
| kg | kilogram(s) |
| mL | milliliter(s) |
| L | liter(s) |
| LCMS | liquid chromatography mass spectrometry |
| °C | degrees Celsius |
| psi | pounds per square inch |
| UV | ultraviolet |

### Examples

Examples 1 provides a general procedure for the conversion of compound I to compound II containing less than 2.5% of compound III. Examples 2 and 3 provide further examples of this process.

### Example 1

General Procedure: Charge nafoxidine hydrochloride (anhydrous, solvent free weight, 1.0 molar equivalent) to the hydrogenation reaction vessel followed by 2B ethanol (13.5 mUg of nafoxidine hydrochloride) and water (1.5 mL/g of nafoxidine hydrochloride). Saturate the reaction mixture with nitrogen and add an appropriate hydrogenation catalyst (0.2 g catalyst/g of nafoxidine hydrochloride). Record the weight of the reaction mixture and pressurize the reaction mixture under a hydrogen atmosphere at a pressure of about 3.40 to about 3.74 atmospheres (344.5 to 378.9 kPa). Heat the reaction mixture until the temperature is in the range of about 48 °C to about 53 °C. The pressure increases as the reaction mixture temperature increases and the pressure is adjusted as required to maintain the pressure in the range of about 3.40 to about 3.74 atmospheres (344.5 to 378.9 kPa). The reaction is monitored after an initial four-hour hydrogenation period and the hydrogenation is allowed to proceed for up to seventy-two hours, typically for four to eight hours, until the reaction is complete with ≤ 1% of the nafoxidine hydrochloride starting material remaining. The reaction mixture is sampled by cooling the reaction mixture to 20 °C to 35 °C, carefully venting the hydrogen and sampling the reaction mixture. After completion of the reaction, the reaction mixture is filtered through Celite 545^{®} to remove the catalyst and the reaction vessel and filter cake are washed with two charges of 2B ethanol (2x2mUg of nafoxidene hydrochloride starting material). The combined filtrate is concentrated in vacuo at 30 °C to 50 °C until the concentration is approximately 2.6 mUg of nafoxidine hydrochloride starting material. Charge toluene (total volume: 20 mUg of nafoxidine hydrochloride starting material divided in 3 lots) to the concentrated filtrate and concentrate in vacuo each time until the concentration is approximately 2.7 mUg of nafoxidine hydrochloride starting material. Cool the resulting slurry to 20 °C to 25 °C and agitate for about 2 hours at this temperature. Collect the product by filtration and wash the filter cake with two lots of toluene (total volume: 4.75 mUg of the nafoxidine hydrochloride starting material). Dry the cis-6-phenyl-5-[4-(2-pyrrolidin-1-yl-ethoxy)-phenyl]-2-methoxy-5,6,7,8-tetrahydronaphthalene hydrochloride product at 40 °C to 50 °C to a constant weight.

The first three entries in Table 1 were carried out according to the above procedure and resulted in the preparation of compound II in 93.7, 95.2 and 93.5 percent yield containing 1.9, 2.4 and 2.4 percent of compound III with total impurities of 2.0, 2.4 and 2.6 percent, respectively.

### Example 2

To a hydrogenation reaction vessel under a nitrogen atmosphere was charged 6.4 kg of 5% palladium on carbon catalyst. In a separate vessel, 32 kg of nafoxidine hydrochloride, 336 L of 2B ethanol and 33.6 L of process water were combined and this mixture was agitated at 15 °C to 25 °C for at least 30 minutes. The resulting solution was transferred into the hydrogenation reaction vessel. The separate vessel was rinsed with 96 L of 2B ethanol and 14.4 L of process water and this rinse was transferred to the hydrogenation reaction vessel. The hydrogenation reaction vessel was then pressurized to 3.07 atmospheres (311.0 kPa) with hydrogen gas and then heated to 48 °C to 53 °C. The pressure in the hydrogenation vessel was then increased to 3.4 to 3.7 atmospheres (344.5 to 374.9 kPa). After hydrogen uptake ceased the hydrogenation reaction was allowed to continue for an additional four hours, then was cooled to 20 °C to 30 °C and the catalyst was filtered off through a Celite^{®} precoated sparkler. The sparkler was washed twice with 64 L of 2B ethanol. The combined filtrate was concentrated in vacuo to a volume of 86 L. 160 L of toluene was added to the filtrate and this mixture was then concentrated in vacuo to a volume of 86 L. 320 L of toluene was then added to the filtrate and the resulting mixture was again concentrated in vacuo to a volume of 86 L. 160 L of toluene was again added to the filtrate and the filtrate was again concentrated in vacuo to a volume of 86 L. The resulting slurry was cooled to 20 °C to 25 °C and granulated for a minimum of two hours with low to medium agitation. The product was then collected by filtration and the filter cake was washed twice with toluene (80 L and 72 L, respectively). The filter cake was then partially dried under nitrogen at ambient temperature to about 80-90% of its original weight.

### Example 3

A hydrogenation reaction vessel was charged with 3.0 g of 5% palladium on carbon catalyst. In a separate vessel, 15.00 g of nafoxidine hydrochloride was combined with 157 mL of 2B ethanol and 15.75 mL of process water and was agitated at 15 °C to 25 °C for a minimum of 30 minutes and the resulting solution was transferred into the hydrogenation reaction vessel. The separate vessel was rinsed with 45 mL 2B ethanol and 6.75 mL process water and this rinse was transferred to the hydrogenation reaction vessel. The hydrogenation reaction vessel was then pressurized to 3.07 atmospheres (311.0 kPa) with hydrogen gas and then heated to 48 °C to 53 °C. The pressure in the hydrogenation vessel was then increased to 3.4 to 3.7 atmospheres (344.5 to 374.9 kPa). After hydrogen uptake ceased the hydrogenation reaction was allowed to continue for an additional four hours, then was cooled to 20 °C to 30 °C and the catalyst was filtered off through a Celite^{®} precoated sparkler. The sparkler was washed twice with 30 mL of 2B ethanol. The combined filtrate was concentrated in vacuo to a volume of 40 mL. 75 mL of toluene was added to the filtrate and this mixture was then concentrated in vacuo to a volume of 40 mL. 150 mL of toluene was then added to the filtrate and the resulting mixture was again concentrated in vacuo to a volume of 40 mL at which point cis-6-phenyl-5-[4-(2-pyrrolidin-1-yl-ethoxy)-phenyl]-2-methoxy-5,6,7,8-tetrahydronaphthalene hydrochloride precipitated from solution. 75 mL of toluene was again added to the filtrate and the filtrate was again concentrated in vacuo to a volume of 40 mL The resulting slurry was cooled to 20 °C to 25 °C and granulated for a minimum of two hours with low to medium agitation. The product was then collected by filtration and the filter cake was washed twice with toluene (38 mL and 34 mL, respectively). The filter cake was then dried at 45 °C to 50 °C for 12 hours to obtain 8.00 g of cis-6-phenyl-5-[4-(2-pyrrolidin-1-yl-ethoxy)-phenyl]-2-methoxy-5,6,7,8-tetrahydronaphthalene hydrochloride as a white solid.

## Claims

1. A process for preparing cis-6-phenyl-5-[4-(2-pyrralidin-1-yl-ethoxy)-phenyl]-2-methoxy-5,6,7,8-tetrahydronaphthalene hydrochloride, wherein said cis-6-phenyl-5-[4-(2-pyrrolidin-1-yl-ethoxy)-phenyl]-2-methoxy-5,6,7,8-tetrahydronaphthalene hydrochloride contains less than 2.5% of 1-{2-[4-(6-methoxy-2-phenyl-naphthalen-1-yl)-phenoxy]-ethyl}-pyrrolidine hydrochloride, the process comprising, in the following order, the steps of:
(a) charging a hydrogenation reaction vessel with an appropriate hydrogenation catalyst and a solution of 1-{2-[4-(6-methoxy-2-phenyl-3,4-dihydronaphthalene-1-yl)phenoxy]ethyl}pyrrolidine hydrochloride in an appropriate solvent;
(b) pressurizing the hydrogenation reaction vessel with hydrogen to a pressure of 137.9 to 689.4 kPa (20 psi to 100 psi) to provide a hydrogenation reaction mixture; (b)
(c) heating the hydrogenation reaction mixture of step (b) at a temperature of 45 °C to 55 °C until 1% or less of the 1-{2-[4-(6-methoxy-2-phenyl-3,4-dihydronaphthalene-1-yl)phenoxy]ethyl}pyrrolidine hydrochloride remains to provide cis-6-phenyl-5-[4-(2-pyrrolidin-1-yl-ethoxy)-phenyl]-2-methoxy-5,6,7,8-tetrahydronaphthalene hydrochloride;
(d) isolating the resulting cis-6-phenyl-5-[4-(2-pyrrolidin-1-yl-ethoxy)-phenyl]-2-methoxy-5,6,7,8-tetrahydronaphthalene hydrochloride; wherein the resulting cis-6-phenyl-5-[4-(2-pyrrolidin-1-yl-ethoxy)-phenyl]-2-methoxy-5,6,7,8-tetrahydronaphthalene hydrochloride contains less than 2.5% of 1-{2-[4-(6-methoxy-2-phenyl-naphthalen-1-yl)-phenoxy]-ethyl}-pyrrolidlne hydrochloride.

2. The process of claim 1, wherein the pressure In step (b) is 137.9 to 413.6 kPa (20 psi to 60 psi).

3. The process of claim 2, wherein the pressure in step (b) is 310.2 kPa to 379.2 kPa (45 psi to 55 psi).

4. The process of claim 3, wherein the hydrogenation catalyst in step (a) is palladium on carbon and the solvent in step (a) is a mixture of ethanol and water.

5. The process of claim 4, wherein the hydrogenation catalyst in step (a) is 5% palladium on activated carbon.

6. The process of claim 5, wherein the solvent in step (a) is a 9:1 mixture of ethanol to water.

7. The process of claim 4, wherein the hydrogenation catalyst in step (a) is 5% palladium on activated carbon and the solvent in step (a) is a 9:1 mixture of ethanol to water.

## Patentansprüche

1. Verfahren zur Herstellung von cis-6-Phenyl-5-[4-(2-pyrrolidin-1-yl-ethoxy)phenyl]-2-methoxy-5,6,7,8-tetrahydronaphthalin-Hydrochlorid, wobei das cis-6-Phenyl-5-[4-(2-pyrrolidin-1-yl-ethoxy)phenyl]-2-methoxy-5,6,7,8-tetrahydronaphthalin-Hydrochlorid weniger als 2,5% 1-{2-[4-(6-Methoxy-2-phenyl-naphthalin-1-yl)phenoxy]ethyl}pyrrolidin-Hydrochlorid enthält, wobei das Verfahren in der folgenden Reihenfolge die Schritte:
(a) Beschicken eines Hydrierungsreaktionsgefäßes mit einem geeigneten Hydrierungskatalysator und einer Lösung von 1-{2-[4-(5-Methoxy-2-phenyl-3,4-dihydronaphthalin-1-yl)phenoxy]ethyl}pyrrolidin-Hydrochlorid in einem geeigneten Lösungsmittel;
(b) unter Druck setzen des Hydrierungsreaktionsgefäßes mit Wasserstoff zu einem Druck von 137,9 bis 689,4 kPa (20 psi bis 100 psi), um ein Hydrierungsreaktionsgemisch bereitzustellen;
(c) Erwärmen des Hydrierungsreaktionsgemisches von Schritt (b) bei einer Temperatur von 45°C bis 55°C bis 1% oder weniger des 1-{2-[4-(6-Methoxy-2-phenyl-3,4-dihydronaphthalin-1-yl)phenoxy]ethyl}pyrrolidin-Hydrochlorid zurückbleiben, um cis-6-Phenyl-5-[4-(2-pyrrolidin-1-yl-ethoxy)phenyl]-2-methoxy-5,6,7,8-tetrahydronaphthalin-Hydrochlorid bereitzustellen;
(d) Isolieren des resultierenden cis-6-Phenyl-5-[4-(2-pyrrolidin-1-yl-ethoxy)phenyl]-2-methoxy-5,6,7,8-tetrahydronaphthalin-Hydrochlorid; wobei das resultierende cis-6-Phenyl-5-[4-(2-pyrrolidin-1-yl-ethoxy)phenyl]-2-methoxy-5,6,7,8-tetrahydronaphthalin-Hydrochlorid weniger als 2,5% 1-{2-[4-(6-Methoxy-2-phenyl-naphthalin-1-yl)phenoxy]-ethyl}pyrrolidin-Hydrochlorid enthält,
umfasst.

2. Verfahren nach Anspruch 1, wobei der Druck in Schritt (b) 137,9 bis 413,6 kPa (20 psi bis 60 psi) ist.

3. Verfahren nach Anspruch 2, wobei der Druck in Schritt (b) 310,2 kPa bis 379,2 kPa (45 psi bis 55 psi) ist.

4. Verfahren nach Anspruch 3, wobei der Hydrierungskatalysator in Schritt (a) Palladium-auf-Kohle ist und das Lösungsmittel in Schritt (a) ein Gemisch aus Ethanol und Wasser ist.

5. Verfahren nach Anspruch 4, wobei der Hydrierungskatalysator in Schritt (a) 5% Palladium-auf-Aktivkohle ist.

6. Verfahren nach Anspruch 5, wobei das Lösungsmittel in Schritt (a) ein 9:1-Gemisch von Ethanol zu Wasser ist.

7. Verfahren nach Anspruch 4, wobei der Hydrierungskatalysator in Schritt (a) 5% Palladium-auf-Aktivkohle ist und das Lösungsmittel in Schritt (a) ein 9:1-Gemisch von Ethanol zu Wasser ist.

## Revendications

1. Procédé de préparation du chlorhydrate de cis-6-phényl-5-[4-(2-pyrrolidin-1-yl-éthoxy)-phényl]-2-méthoxy-5,6,7,8-tétrahydronaphtalène, dans lequel ledit chlorhydrate de cis-6-phényl-5-[4-(2-pyrrolidin-1-yl-éthoxy)-phényl]-2-méthoxy-5,6,7,8-tétrahydronaphtalène contient moins d'environ 2,5 % de chlorhydrate de 1-{2-[4-(6-méthoxy-2-phényl-naphtalèn-1-yl)-phénoxy]-éthyl}-pyrrolidine, le procédé comprenant, dans l'ordre suivant, les étapes de :
(a) chargement d'un réacteur d'hydrogénation avec un catalyseur d'hydrogénation approprié et une solution de chlorhydrate de 1-{2-[4-(6-méthoxy-2-phényl-3,4-dihydro-naphtalène-1-yl)phénoxy]éthyl}pyrrolidine dans un solvant approprié ;
(b) mise sous pression du réacteur d'hydrogénation avec de l'hydrogène jusqu'à une pression comprise entre 137,9 et 689,4 kPa (entre 20 livres/pouce² et 100 livres/pouce²) pour réaliser un mélange réactionnel d'hydrogénation ;
(c) chauffage du mélange réactionnel d'hydrogénation de l'étape (b) à une température comprise entre 45°C et 55°C jusqu'à ce qu'il ne reste que 1 % ou moins du chlorhydrate de 1-{2-[4-(6-méthoxy-2-phényl-3,4-dihydronaphtalène-1-yl)phénoxy]éthyl}pyrrolidine pour livrer le chlorhydrate de cis-6-phényl-5-[4-(2-pyrrolidin-1-yl-éthoxy)-phényl]-2-méthoxy-5,6,7,8-tétrahydronaphtalène ;
(d) l'isolement du chlorhydrate de cis-6-phényl-5-[4-(2-pyrrolidin-1-yl-éthoxy)-phényl]-2-méthoxy-5,6,7,8-tétrahydronaphtalène résultant ; le chlorhydrate de cis-6-phényl-5-[4-(2-pyrrolidin-1-yl-éthoxy)-phényl]-2-méthoxy-5,6,7,8-tétrahydronaphtalène résultant renfermant moins de 2 , 5 % de chlorhydrate de 1-{2-[4-(6-méthoxy-2-phényl-naphtalèn-1-yl)-phénoxy]-éthyl}-pyrrolidine.

2. Procédé selon la revendication 1, dans lequel la pression à l'étape (b) est comprise 137,9 et 413,6 kPa (entre 20 livres/pouce² et 60 livres/pouce²) .

3. Procédé selon la revendication 2, dans lequel la pression à l'étape (b) est comprise entre 310,2 kPa et 379,2 kPa (entre 45 livres/pouce² et 55 livres/pouce²).

4. Procédé selon la revendication 3, dans lequel le catalyseur d'hydrogénation à l'étape (a) est du palladium sur carbone et le solvant à l'étape (a) est un mélange d'éthanol et d'eau.

5. Procédé selon la revendication 4, dans lequel le catalyseur d'hydrogénation à l'étape (a) est du palladium à 5 % sur carbone activé.

6. Procédé selon la revendication 5, dans lequel le solvant à l'étape (a) est un mélange 9:1 d'éthanol et d'eau.

7. Procédé selon la revendication 4, dans lequel le catalyseur d'hydrogénation à l'étape (a) est du palladium à 5 % sur carbone activé et le solvant à l'étape (a) est un mélange 9:1 d'éthanol et d'eau.
